(19) Europäisches Patentamt
European Patent Office
Office européen des brevets

(11) Numéro de publication : **0 024 224**
**B1**

(12) # FASCICULE DE BREVET EUROPÉEN

(45) Date de publication du fascicule du brevet : **19.05.82**

(51) Int. Cl.³ : **C 07 C 29/124**, C 07 C 31/38, C 07 C 67/00, C 07 C 69/003

(21) Numéro de dépôt : **80401108.8**

(22) Date de dépôt : **25.07.80**

(54) **Procédé de préparation des 1,1,2,2-tétrahydro perfluoro alcanols et des esters de ces alcools.**

(30) Priorité : **08.08.79 FR 7920280**

(43) Date de publication de la demande : **25.02.81 (Bulletin 81/08)**

(45) Mention de la délivrance du brevet : **19.05.82 Bulletin 82/20**

(84) Etats contractants désignés : **BE CH DE FR GB LI NL SE**

(56) Documents cités :
**DE - A - 2 318 677**
**US - A - 3 182 008**

**JOURNAL OF THE CHEMICAL SOCIETY CHEMICAL COMMUNICATIONS, vol. 21, 1er novembre 1978 publiée par « The Chemical Society » Londres GB R.C. CAMBIS et al. : « Oxidative displacement of iodine from vicinal iodocarboxylates and alkyl iodides », page 919**

**JOURNAL OF THE CHEMICAL SOCIETY PERKIN TRANSACTION I, vol. n° 3, mars 1980 publiée par « The Chemical Society » Londres GB R.C. CAMBIE et al. : « Oxidative displacement of Hypervalent iodine from alkyliodides », pages 822-827**

(73) Titulaire : **P C U K PRODUITS CHIMIQUES UGINE KUHLMANN**
**Service Propriété Industrielle Tour Manhattan**
**F-92087 Paris La Défense 2 Cedex 21 (FR)**

(72) Inventeur : **Foulletier, Louis**
**5 Chemin Croix-Berthet**
**F-69600 Oullins (FR)**
Inventeur : **Lantz, André**
**Domaine de la Hêtraie**
**F-69390 Vernaison (FR)**

(74) Mandataire : **Bernard, Nicole et al**
**PCUK Produits Chimiques Ugine Kuhlmann Service Propriété Industrielle Tour Manhattan - Cedex 21**
**F-92087 Paris la Défense 2 (FR)**

**EP 0 024 224 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

# Procédé de préparation des 1,1,2,2-tetrahydro perfluoro alcanols et des esters de ces alcools

La présente invention concerne un procédé de préparation des 1,1,2,2-tétrahydro perfluoro alcanols et des esters carboxyliques de ces alcools. Ces produits répondent aux formules générales :

$$R_FCH_2CH_2OH$$
et
$$R_FCH_2CH_2OCOR$$

où $R_F$ représente une chaîne perfluorée, droite ou ramifiée contenant de 1 à 20 atomes de carbone et R représente un atome d'hydrogène ou un radical hydrocarboné aromatique ou aliphatique.

Ces produits ont été obtenus jusqu'à présent selon le procédé décrit dans le brevet français n° 1 380 579 par réaction des iodures $R_FCH_2CH_2I$ avec de l'oléum suivie d'une hydrolyse des sulfates formés. Cependant, ce procédé présente l'inconvénient de fournir des quantités importantes de diesters sulfuriques qui sont difficilement hydrolysables. Selon le procédé décrit dans le brevet américain 3 239 557 il est possible d'obtenir les esters $R_FC_2H_4OCOR$ par réaction classique des iodures $R_FC_2H_4I$ avec des sels des acides carboxyliques RCOOH. Les rendements ne sont cependant pas très bons car il se forme des quantités plus ou moins importantes d'oléfines $R_FCH = CH_2$.

Les alcools peuvent aussi être obtenus selon le procédé du brevet français 2 096 179 qui consiste à préparer les nitrates $R_FCH_2CH_2ONO_2$ par réaction des iodures $R_FCH_2CH_2I$ avec l'acide nitrique et à hydrogéner ces nitrates en alcool. Ce procédé présente toutefois l'inconvénient de nécessiter deux étapes réactionnelles dont la dernière doit être réalisée sous une pression élevée d'hydrogène.

Le brevet français 2 180 113 décrit un procédé de fabrication de mélanges d'alcool $R_FC_2H_4OH$ et de formiates $R_FC_2H_4$ OCOH par réaction à haute température des iodures $R_FC_2H_4I$ avec de la diméthyl formamide en présence d'un peu d'eau. Ce procédé présente l'inconvénient d'exiger des conditions de réaction très sévères et de fournir comme sous-produit de l'oléfine $R_FCH{=}CH_2$, ce qui diminue d'autant le rendement. Une bonne sélectivité en alcool et formiate ne peut être obtenue qu'en utilisant des quantités très importantes de diméthyl formamide.

Le brevet américain 3 182 008 décrit l'hydroxylation d'une chaîne hydrocarbonée par un peracide pour obtenir des alcools mais lorsque ce procédé est appliqué à des dérivés aliphatiques halogénés il se forme des dérivés hydroxy halogénés et non des alcools simples.

Le Journal of the Chem. Soc., Chem. Communications *21* p. 919 décrit la réaction d'un peracide sur un iodure d'alkyle conduisant, suivant le solvant utilisé, à un alcool ou un mélange d'ester et d'alcool et éventuellement de cétone. Cependant la présence dans la molécule $R_FCH_2CH_2I$ d'un groupement perfluoré fortement électroattractif à proximité de l'atome d'iode devrait conduire d'après la littérature, par exemple Journal of Am. Chem. Soc. juillet 1978 p. 4 888, à la formation de l'oléfine correspondante avec l'élimination d'acide iodhydrique.

Or le procédé de la présente invention permet de préparer des esters $R_FCH_2CH_2OCOR$ et/ou des alcools $R_FCH_2CH_2OH$ à partir des iodures fluorés $R_FC_2H_4I$ par réaction avec des acides percarboxyliques $RCO_3H$. Il a en effet été constaté que lorsqu'on mélange un iodure $R_FCH_2CH_2I$ avec un peracide $RCO_3H$, il y a formation immédiate d'iode et le produit fluoré est transformé en alcool $R_FCH_2CH_2OH$, en esters $R_FCH_2CH_2OCOR$ ou en un mélange des deux. Les proportions relatives d'alcool et d'ester dépendent principalement de la nature du peracide et des conditions opératoires (nature du solvant, teneur en eau du milieu, température, rapport molaire des réactifs...).

Les peracides utilisables sont par exemple les acides perbenzoïque, p. nitroperbenzoïque, chloroperbenzoïque, perphtalique mais on préfère en général les peracides aliphatiques, plus facilement accessibles, comme les acides performique, peracétique, perpropionique ou perbutyrique. Ces peracides peuvent être obtenus selon les méthodes de préparation connues pour ces produits. L'une de ces méthodes de préparation, particulièrement simple, consiste à utiliser un mélange de peroxyde d'hydrogène et d'acide carboxylique qui fournit l'acide percarboxylique selon la réaction équilibrée :

$$RCOOH + H_2O_2 \leftrightarrow RCO_3H + H_2O$$

Selon la valeur du rapport $RCOOH/H_2O_2$, la nature du radical R, la quantité d'eau introduite avec les réactifs et selon la température on obtient un certain taux de transformation du peroxyde d'hydrogène en peracide. Ainsi dans le cas de l'acide formique, avec du peroxyde d'hydrogène à 70 % à 20 °C le taux de transformation à l'équilibre du peroxyde d'hydrogène en acide performique est de 70 % avec un rapport molaire $HCOOH/H_2O_2$ de 3, de 82 % pour un rapport molaire de 5 et de 95 % pour un rapport molaire de 10. La vitesse de formation du peracide à partir de l'acide et du peroxyde d'hydrogène dépend de la nature de l'acide mis en jeu et la réaction peut en général être accélérée par l'introduction de faibles quantités d'un acide fort tel que $H_2SO_4$. Dans le cas des acides organiques forts comme par exemple HCOOH ou $CF_3COOH$ l'équilibre peut être atteint rapidement sans catalyseur. Les acides percarboxyliques obtenus selon cette méthode conviennent parfaitement au procédé de l'invention, même si la transformation du peroxyde d'hydrogène en peracide n'est pas quantitative et si ces mélanges contiennent encore du

peroxyde d'hydrogène non transformé.

Des peracides obtenus par tout autre procédé peuvent également être utilisés pour la présente invention.

La réaction de l'invention est de préférence conduite en milieu solvant. Le solvant peut être soit l'acide carboxylique à partir duquel le peracide a été préparé ($HCOOH$, $CH_3COOH$, $CH_3CH_2COOH$...) soit un autre solvant inerte vis-à-vis des peracides comme les carbures aromatiques, les solvants halogénés ($CH_2Cl_2$, $CHCl_3$, dichloréthane, trichloro trifluoro éthane...) certains hydrocarbures ou certains éthers (dioxanne)...

La réaction peut être réalisée en introduisant l'iodure $R_FCH_2CH_2I$ dans le peracide mais, en vue de réduire au maximum la décomposition du peracide, on a intérêt à introduire le peracide dans l'iodure. La température de réaction n'est pas critique et celle-ci peut être réalisée dans une gamme de température assez large, mais on préfère en général la réaliser à des températures voisines de la température ambiante (15 à 45 °C).

Selon les quantités engagées de peracide, l'iode de l'iodure fluoré peut se retrouver à un degré d'oxydation plus élevé que celui de l'iode élémentaire et on peut en particulier former les acides hypoiodeux et iodique. Cette oxydation supplémentaire consomme évidemment des quantités importantes de peracides ou de peroxyde d'hydrogène, mais il est possible de réduire cet inconvénient en fixant l'iode au fur et à mesure de sa formation. L'une des méthodes permettant de capter l'iode consiste à réaliser la réaction en présence d'un carbure aromatique (benzène, toluène, xylène) qui se transforme partiellement en un dérivé aromatique iodé selon la réaction :

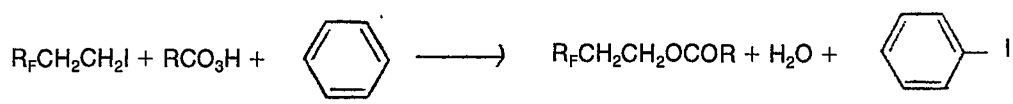

Les produits fluorés formés sont insolubles dans l'eau et peuvent en général être séparés par simple décantation après dilution du milieu réactionnel par de l'eau. Cette séparation est de préférence réalisée après réduction des dérivés iodés par un réducteur classique tel que le sulfite de sodium par exemple. Lorsque la réaction est effectuée en présence d'un solvant non miscible à l'eau, ce solvant peut être séparé des produits fluorés par toute technique appropriée par exemple par distillation.

La transformation de l'iodure $R_FCH_2CH_2I$ en alcools et esters se fait avec des sélectivités excellentes et il n'a pas été observé de formation de sous-produits. Il se forme en particulier très peu d'oléfine $R_FCH = CH_2$, ce sous-produit qui se forme en général en quantités importantes au cours de la plupart des autres procédés de fabrication, et qui en abaisse les rendements de façon notable.

Les esters fluorés $R_FCH_2CH_2OCOR$ et les alcools fluorés $R_FCH_2CH_2OH$ préparés selon ce procédé sont des matières premières intéressantes pour la fabrication d'agents tensioactifs et de produits hydrophobes et oléophobes. Ces produits peuvent en particulier être transformés facilement en esters acryliques ou méthacryliques qui permettent de préparer par polymérisation des agents hydrophobes et oléophobes pour les matières textiles, le cuir ou le papier. Les mélanges d'esters $R_FCH_2CH_2OCOR$ et d'alcools $R_FCH_2CH_2OH$ peuvent être également transformés en produits intéressants. Ainsi un mélange d'alcool $R_FCH_2CH_2OH$ et de formiate $R_FCH_2CH_2OCOH$ peut être transformé en acrylate par transestérification avec un acrylate de méthyle par exemple. Le mélange d'alcool et d'ester peut aussi être transformé entièrement en alcool par hydrolyse de l'ester, par saponification ou par transestérification avec du méthanol par exemple.

Les exemples suivants illustrent l'invention de façon non limitative :

Exemple 1

Dans un réacteur contenant 7,9 g d'acide p. nitro perbenzoïque (0,043 mole) et 50 ml de chlorure de méthylène, on introduit 12 g de $C_6F_{13}CH_2CH_2I$ (0,025 mole) en maintenant la température entre 25 et 30 °C par refroidissement extérieur avec de l'eau froide. Le mélange est maintenu à cette température, sous agitation, pendant 3 heures. On introduit ensuite 100 ml d'eau et on analyse par chromatographie en phase gazeuse la phase liquide. Celle-ci contient en dehors du chlorure de méthylène un mélange de $C_6F_{13}CH_2CH_2OH$ et de $C_6F_{13}CH_2CH_2I$ dans les proportions suivantes :

$C_6F_{13}CH_2CH_2OH$ : 52 %
$C_6F_{13}CH_2CH_2I$ : 48 %

Exemple 2

On introduit dans un réacteur agité 92 g d'acide formique, 10 g de peroxyde d'hydrogène à 70 % et 1 g d'acide sulfurique et on maintient ce mélange sous agitation pendant 1 heure à 20 °C, en vue de préparer de l'acide performique.

On ajoute ensuite le mélange résultant en 1 h 40 à un mélange de 48 g de $C_6F_{13}CH_2CH_2I$ et de 20 g

d'acide formique, en maintenant la température à 40 °C pendant toute l'opération. Le milieu réactionnel est encore maintenu à 40 °C pendant une heure après la fin de l'introduction, puis on introduit 100 ml d'eau et on décolore le milieu par addition de sulfite de sodium. On décante ensuite la phase inférieure et on la lave 2 fois avec 50 ml d'eau. On récupère ainsi 41 g de produit contenant :

1 % $C_6F_{13}CH_2CH_2OH$
44 % $C_6F_{13}CH_2CH_2I$
53 % $C_6F_{13}CH_2CH_2OCOH$

Le mélange réactionnel a été analysé par chromatographie en phase gazeuse, après étalonnage avec des échantillons des différents produits purs.

Exemple 3

On introduit dans un réacteur agité 46 g d'acide formique, 5 g de peroxyde d'hydrogène à 70 % et 0,5 g d'acide sulfurique et on maintient ce mélange sous agitation pendant une heure à 20 °C.

On coule ensuite cette solution en 1 heure dans un mélange de 24 g de $C_6F_{13}CH_2CH_2I$ et de 20 ml de chlorure de méthylène en maintenant la température à 30 °C. Le milieu réactionnel est encore maintenu à 30 °C pendant 1 heure après la fin de l'introduction des réactifs, puis on coule 50 ml d'eau et on ajoute du sulfite de sodium jusqu'à décoloration complète de l'iode. On décante la phase inférieure et on la lave avec 50 ml d'eau. On évapore ensuite le chlorure de méthylène et on récupère 19 g de produit fluoré contenant :

3 % $C_6F_{13}CH_2CH_2OH$
35 % $C_6F_{13}CH_2CH_2I$
58 % $C_6F_{13}CH_2CH_2OCOH$

Exemple 4

On répète l'exemple 2 mais en introduisant le peracide obtenu à partir de

| | |
|---|---|
| HCOOH : | 92 g |
| $H_2O_2$ 70 % : | 10 g |
| $H_2SO_4$ : | 1 g |

| | |
|---|---|
| à un mélange de $C_6F_{13}CH_2CH_2I$ : | 24 g |
| HCOOH : | 20 g |

Après traitement on obtient 19 g de produit contenant :

| | |
|---|---|
| $C_6F_{13}CH=CH_2$ : | 0,1 % |
| $C_6F_{13}CH_2CH_2OH$ : | 2,6 % |
| $C_6F_{13}CH_2CH_2I$ : | 15 % |
| $C_6F_{13}CH_2CH_2OCOH$ : | 80 % |

Exemple 5

On prépare une solution d'acide performique en mélangeant 92 g d'acide formique et 10 g de peroxyde d'hydrogène à 70 % et on maintient ce mélange à 20 °C pendant 1 h 30. On coule ensuite sous agitation ce produit en 2 heures dans un mélange de 48 g de $C_6F_{13}CH_2CH_2I$ et de 20 g d'acide formique en maintenant la température à 25-30 °C, on laisse reposer le mélange obtenu à 25 °C pendant 1 heure, puis on le traite à l'eau et au sulfite. On obtient 40 g de produit contenant :

| | |
|---|---|
| $C_6F_{13}CH_2CH_2OH$ : | 7 % |
| $C_6F_{13}CH_2CH_2I$ : | 14,8 % |
| $C_6F_{13}CH_2CH_2OCOH$ : | 76,5 % |

Exemple 6

On répète l'exemple 2, mais la solution de peracide est coulée dans le $C_6F_{13}CH_2CH_2I$ en maintenant la température de ce dernier à 60 °C. Après traitement, on récupère 38 g de produit contenant ;

| | |
|---|---|
| $C_6F_{13}CH_2CH_2OH$ : | 1,3 % |
| $C_6F_{13}CH_2CH_2I$ : | 35 % |
| $C_6F_{13}CH_2CH_2OCOH$ : | 62 % |

Exemple 7

On répète l'exemple 2, mais en remplaçant le $C_6F_{13}CH_2CH_2I$ par 37,5 g de $C_4F_9CH_2CH_2I$. On obtient ainsi, après traitement, 28 g de produit contenant :

| | |
|---|---|
| $C_4F_9CH_2CH_2OH$ : | 2 % |
| $C_4F_9CH_2CH_2I$ : | 36 % |
| $C_4F_9CH_2CH_2OCOH$ : | 60 % |

Exemple 8

On répète l'exemple 2 mais en remplaçant l'iodure ayant un $R_F$ 25 à 6 atomes de carbone par 53,7 g d'un $R_FCH_2CH_2I$ constitué d'un mélange des différents homologues de $C_4F_9CH_2CH_2I$ à $C_{18}F_{37}CH_2CH_2I$ de composition pondérale suivante ;

| | |
|---|---|
| $C_4F_9CH_2CH_2I$ : | 0,9 % |
| $C_6F_{13}CH_2CH_2I$ : | 51 % |
| $C_8F_{17}CH_2CH_2I$ : | 28,2 % |
| $C_{10}F_{21}CH_2CH_2I$ : | 11,5 % |
| $C_{12}F_{25}CH_2CH_2I$ : | 4,4 % |
| $C_{14}F_{29}CH_2CH_2I$ : | 1,7 % |
| $C_{16}F_{33}CH_2CH_2I$ : | 0,6 % |
| $C_{18}F_{37}CH_2CH_2I$ : | 0,2 % |

Le poids moléculaire moyen de ce produit est voisin de 534.

Après réaction et traitement comme indiqués dans l'exemple 2, on obtient 45 g de produit contenant environ :

| | |
|---|---|
| $R_FCH_2CH_2OH$ : | 2 % |
| $R_FCH_2CH_2I$ : | 50 % |
| $R_FCH_2CH_2OCOH$ : | 46 % |

Exemple 9

On prépare une solution d'acide peracétique en maintenant pendant 2 h 30 à 20 °C un mélange de 50 g d'acide acétique, 5 g de peroxyde d'hydrogène à 70 % et 0,5 g d'acide sulfurique. Le mélange obtenu est ensuite ajouté en 1 h 30 à 24 g de $C_6F_{13}CH_2CH_2I$, la température étant maintenue à 30 °C pendant toute la durée de l'addition. On maintient encore le mélange réactionnel à 30 °C pendant 1 heure, puis on le traite avec 100 ml d'eau et une solution de sulfite de sodium en quantité suffisante pour le décolorer, et on obtient après décantation et lavage à l'eau, 21 g de produit fluoré contenant d'après l'analyse chromatographique ;

| | |
|---|---|
| $C_6F_{13}CH = CH_2$ : | 0,8 % |
| $C_6F_{13}CH_2CH_2OH$ : | 15 % |
| $C_6F_{13}CH_2CH_2I$ : | 45 % |
| $C_6F_{13}CH_2CH_2OCOH$ : | 37 % |

**Revendications**

1. Procédé de préparation d'alcools $R_FCH_2CH_2OH$ et d'esters $R_FCH_2CH_2OCOR$ où $R_F$ représente une chaîne perfluorée, droite ou ramifiée contenant de 1 à 20 atomes de carbone et R représente un atome d'hydrogène ou un radical hydrocarboné, aromatique ou aliphatique, consistant à faire réagir un iodure $R_FCH_2CH_2I$ avec un peracide $RCO_3H$.
2. Procédé selon la revendication 1 où la réaction a lieu entre 15 et 45 °C.
3. Procédé selon d'une des revendications 1 ou 2 où la réaction a lieu en milieu solvant.
4. Procédé selon l'une des revendications 1 à 3 où le peracide est l'acide performique.
5. Procédé selon l'une des revendications 1 à 3 où le peracide est l'acide peracétique.

**Claims**

1. Process for the preparation of alcohols $R_FCH_2CH_2OH$ and esters $R_FCH_2CH_2OCOR$, where $R_F$ represents a linear or branched perfluorinated chain containing from 1 to 20 carbon atoms and R

represents a hydrogen atom or an aromatic or aliphatic hydrocarbon radical, which comprises reacting an iodide $R_FCH_2CH_2I$ with a per-acid $RCO_3H$.

2. Process according to Claim 1, wherein the reaction takes place at between 15 and 45 °C.
3. Process according to one of Claims or 2, wherein the reaction takes place in a solvent medium.
4. Process according to one of Claims 1 to 3, wherein the per-acid is performic acid.
5. Process according to one of Claims 1 to 3, wherein the per-acid is peracetic acid.

**Ansprüche**

1. Verfahren zur Herstellung von Alkoholen $R_FCH_2CH_2OH$ und Estern $R_FCH_2CH_2OCOR$, in denen $R_F$ eine gerade oder verzweigte perfluorierte Kette mit 1 bis 20 Kohlenstoffatomen und R ein Wasserstoffatom oder einen aromatischen oder aliphatischen Kohlenwasserstoffrest bedeutet, dadurch gekennzeichnet, daß man ein Jodid $R_FCH_2CH_2I$ mit einem Peracid $RCO_3H$ umsetzt.
2. Verfahren nach Anspruch 1, bei dem die Reaktion zwischen 10 und 45 °C stattfindet.
3. Verfahren nach einem der Ansprüche 1 oder 2, wobei die Reaktion in einem Lösungsmittel erfolgt.
4. Verfahren nach einem der Ansprüche 1 bis 3, bei dem die Persäure Perameisensäure ist.
5. Verfahren nach einem der Ansprüche 1 bis 3, bei dem die Persäure Peressigsäure ist.